# EUROPEAN PATENT APPLICATION

(11) **EP 2 119 724 A1**
(43) Date of publication of application: **18.11.2009**
(21) Application number: 09010808.5
(22) Date of filing: 02.05.2006
(51) Int. Cl.: C07K 1/00, C07K 7/23, C07K 14/655, C07K 14/585, C07K 7/18, C07K 14/575, C07K 14/16, C07K 14/645, C07K 14/435, A61K 38/04

(54) **Solid-phase process foor the preparation of goserelin**

(30) Priority: 03.05.2005 US 677582 P
(62) Divisional of application: 06759010.9
(71) Applicant: Novetide, Ltd., 26111 Haifa Bay (IL)
(72) Inventor: Ivchenko, Alexander, Keryat Ata 28076 (IL); Alon, Hagi, D.N. 25125 (IL); Elster, Shai, Haifa 32811 (IL); Shushan, Shimon, Karmiel, 21926 (IL); Eidelman, Chaim, D.N. Oshrat 25167 (IL); Tovi, Avi, Zurit 20104 (IL); Gadi, Tehila, Kfar Vradim 25147 (IL); Bar-Oz, Leah, 27070 Keryat Bialik (IL); Alterman, Eleonora, 34760 Haifa (IL); Zaovi, Gil, 27070 Keryat Bialik (IL); Butilca, Gabriel-Marcus, Karmiel 20100 (IL)
(74) Representative: King, Lawrence

(57) **Abstract**

The invention relates to a method for the preparation of goserelin by a combination of solid-phase synthesis and post assembly solution phase synthesis. The invention also relates to pure goserelin acetate.

## Description

### Related Applications

This application claims benefit of U.S. Provisional Patent Application No. 60/677,582, filed May 3, 2005, incorporated herein by reference.

### Field of the Invention

The present invention relates to a method of preparing a peptide which is a C-terminal amide derivative and to products thereof.

### Background of the Invention

Peptide synthesis may be either solid-phase synthesis (SPPS) or solution-phase synthesis and generally proceeds from the C-terminus to N-terminus. There are several groups of peptide and peptidomimetic compounds characterized by derivatization at the carboxy terminus of the peptide chain.

Within the category of peptides derivatized at the C-terminus, one of the most important families of pharmaceutical products is the LH-RH analogs. This family consists of various peptides such as Leuprolide, Triptorelin, Buserelin, Goserelin, and other analogues.

Leuprolide acetate is a synthetic nonapeptide analog of naturally occurring gonadotropin-releasing hormone (GnRH or LH-RH). Its chemical name is 5-oxo-L-prolyl-L-histidyl-L-tryptophyl-L-seryl-L-tyrosyl-D-leucyl-L-leucyl-L-arginyl-N-ethyl-L-prolinamide, and its primary sequence is: pGlu-His-Trp-Ser-Tyr-D-Leu-Leu-Arg-Pro-NHEt (SEQ. ID. NO. 1). Leuprolide possesses greater potency than the natural hormone. In males, Leuprolide acts by inhibiting the production of testosterone, which may play a significant role in prostate cancer growth. In females, it reduces the production of estrogen and so is used in the management of endometriosis and uterine fibroids. In children, it is used in the treatment of precocious puberty. It is marketed in the United States as an implantation under the name VIADUR® or as an injection under the name LEUPRON DEPOT®.

It was found that even a minor modification of the amino acids in the peptide significantly diminishes the physiological activity of the peptide (Schally et al, Biochem. Biophys. Res. Commun., 4, 366 (1972)).

The synthesis of derivatized peptides is usually done by a solid phase peptide synthesis (SPPS) or a solution phase synthesis. The SPPS usually involves the use of a resin on which the derivatized peptide is built on. The solution phase synthesis is usually based on fragment condensation.

The SPPS is described in BE 841180 and U.S. Patent No. 4,002,738. By this procedure, proline carrying as a blocking group the t-butyloxy-carbonyl substituent (Boc- ) on the amino group is esterified by combination with a chloromethylated divinylbenzene-styrene copolymer (Merrifield resin), using the method described by Stewart, et al. in "SOLID PHASE PEPTIDE SYNTHESIS", (published in 1969 by Freeman & Company). The synthesis is continued sequentially in an automatic synthesizer, applying Boc chemistry for the synthesis of the desired nonapeptide. Deprotection of the Boc group was effected by 4N hydrochloric acid/dioxane. Coupling was achieved by the use of dicyclohexylcarbodiimide in dichloromethane at a 2.9 fold excess. Finally, the peptide resin obtained by this procedure was suspended in 200 ml of 5% triethylamine/methanol and 100 ml of distilled ethylamine was added thereto. After 24 hours, the resin was removed by filtration and the solution evaporated to yield a solid. The solid was dissolved in glacial acetic acid and purified on a silica gel column to obtain tri-protected nonapeptide (with protective groups at Ser, Tyr, and Arg). Final deprotection was done by anhydrous hydrogen fluoride. The crude product was finally purified by chromatography on a Sephadex G-25 column (marketed by Pharmacia of Uppsala, Sweden).

Another example of SPPS is found in U.S. Patent No. 4,005,063.

In general, the peptides can be made by using the SPPS described by Merrifield in J. Am. Chem. Soc., 85, 2149 (1963). More particularly, N-blocked proline is esterified to a chloromethylated divinylbenzene-styrene copolymer. After deblocking, N^{γ}-blocked arginine carrying a labile protective group on the imino-N is coupled to the now free imino group of the proline ester and, after deblocking, this sequence of coupling and deblocking steps is repeated with other amino acids in the sequence of the desired peptide. All of the amino acids are used in their L-form except for the amino acid identified as D-amino acid in the formula. After all of these amino acids are linked in the above sequence with the arginine, tyrosine, serine and optionally the histidine carrying protecting groups, the nonapeptide is removed from the resin via transesterification/ammonolyzis whereby the resin link is replaced by the ethylamide terminus. Subsequent treatment in known fashion removes all the protective groups, producing the peptide in substantially pure form and acceptable yield.

European Patent Application EP 0518656A2 describes a SPPS of the Goserelin sequence on a resin through a linkage which is labile to hydrazine. Cleavage of the peptide from the resin results in the hydrazide derivative, which can be converted into the aza-Gly terminal residue. The protection of side chains is achieved by use of the following protecting groups: BrZ for Tyr, Fmoc for His, and tBu for D-Ser at the 6 position, avoiding protection of the Ser at position 4.

Another European Patent Application, EP 0518655A2, describes a SPPS starting with a resin preloaded with the AzaGly building unit. No protection for the Tyr and Ser side chains at the 4 position is used. The final peptide is treated with hydrazine to hydrolyze possible side products with acylated amino-acid side chains which are incorporated in free form.

European Patent Application EP 1179537, describes a SPPS of a peptide sequence which is carried out sequentially using super acid-labile protecting groups and another type of super acid labile resin in such a way that the peptide could be removed from the resin while keeping side chain protecting groups that can be removed later by another acidic treatment. The C-terminal group such as aza-glycine or ethylamine is attached to the protected peptide chain via a regular amide formation procedure. The main disadvantage of this method is the necessity of applying unique and expensive protection strategies.

Another methodology is a solution phase synthesis, based on fragment condensation, as described by International Patent Publication WO 99/07874. By this method, the required peptide can be obtained by reacting a peptide fragment represented by the following general formula pGlu-His-Trp-OR₁ (wherein R₁ represents lower alkyl) with another peptide fragment represented by the following general formula H-Ser-Tyr-X-Leu-Arg-Pro-Y in the presence of chymotrypsin or a chymotrypsin-like enzyme.

Another variation of the fragment condensation method is disclosed in U.S. Patent No. 4,008,209. In this patent, a nonapeptide amide derivative is produced by a method in which a reagent (A) -- L-pyroglutamic acid or a peptide fragment which has an L-pyroglutamic acid unit (i.e., (Pyr)Glu-) at its N-terminal end and at the same time which, from thereon, comprises the desired amino acid sequence -- is condensed with a reagent (B) -- an amine component which corresponds to the balance of the nonapeptide amide derivative --, the two reagents (A) and (B) being optionally protected by a protecting group or groups, and then the protecting group or groups, if any, are removed.

In another example of the same solution phase synthetic approach (Russian Patent Application RU 2074191) the synthesis is performed according to a 2+[2+(4+1)] fragmentation scheme, applying Cbz chemistry and a side chain unprotected Arg residue.

In another example (International Patent Publication WO 97/48726), the peptide chain is built by a 2+4+3 fragment coupling strategy. Cbz protecting chemistry is applied and one of the intermediates is purified by crystallization, while the final peptide is purified by ion exchange chromatography.

U.S. Patent No. 4,100,274 describes a method of obtaining Goserelin by means of the condensation of three pre-formed fragments which contain -NO₂ as the protecting group for arginine and -Bzl as the protecting group for tyrosine, both of which are labile to hydrogenolysis. In this method, the azaglycine residue is introduced into the C-terminal tripeptide, which is then coupled to Z-Tyr(Bzl)-D-Ser(tBu)-Leu-N_{3,} to give a fragment which, once the Z group is removed, couples to Pyr-His-Trp-Ser-N₃ to give Goserelin. This last reaction is carried out with all the side chains unprotected with the exception of that belonging to D-Ser(tBu).

### Summary of the Invention

In one embodiment, the present invention provides a method of preparing a peptide which is a C-terminal amide derivative, comprising: providing amino acid, protected or non-protected, attached in its C-terminal to a super-acid labile resin; coupling said amino acid, with another amino acid, protected or non-protected, in the presence of a coupling reagent; repeating the coupling step to obtain a peptide, wherein the peptide is protected with at least one protecting group which remains on the peptide upon its cleavage from the resin; cleaving said protected peptide from the resin by admixing with a mild acidic solution; and amidating the obtained protected peptide with a suitable amine.

In another embodiment, the present invention provides Leuprolide acetate containing less than about 0.1% D-Ser⁴-Leuprolide.

In yet another embodiment, the present invention provides Leuprolide acetate containing less than about 0.2% D-His²-Leuprolide.

In one embodiment, the present invention provides Leuprolide acetate containing less than about 0.1 % D-pGlu¹-Leuprolide

In another embodiment, the present invention provides Leuprolide acetate containing not more than about 0.1 % of any other impurity.

In yet another embodiment, the present invention provides Goserelin acetate containing less than about 0.5% of any other impurity.

In one embodiment, the present invention provides Triptorelin acetate containing less than about 0.5% of any other impurity.

In another embodiment, the present invention provides pGlu-His(Trt)-Trp-Ser(tBu)-Tyr(tBu)-D-Leu-Leu-Arg(Pbf)-Pro-OH (SEQ. ID. NO. 1) (protected Leuprolide precursor).

In yet another embodiment, the present invention provides pGlu-His-Trp-Ser-Tyr(Bzl)-D-Ser(tBu)-Leu-Arg(NO2)-Pro- (SEQ. ID. NO. 4) (protected Goserelin precursor).

In one embodiment, the present invention provides pGlu-His(Trt)-Trp-Ser(tBu)-Tyr(tBu)-D-Trp-Leu-Arg(Pbf)-Pro-Gly-OH (SEQ. ID. NO. 2) (protected Triptorelin precursor).

In yet another embodiment, the present invention provides peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate having a purity of at least about 99.0% as determined by HPLC method.

In one embodiment, the present invention provides a pharmaceutical composition comprising peptide acetate made by one of the processes of the present invention and at least one pharmaceutically acceptable excipient.

In another embodiment, the present invention provides a process for preparing a pharmaceutical formulation comprising combining peptide acetate made by one of the processes of the present invention, with at least one pharmaceutically acceptable excipient.

In yet another embodiment, the present invention provides the use of peptide acetate made by one of the processes of the present invention for the manufacture of a pharmaceutical composition.

In one embodiment, the present invention provides a process for preparing peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate comprising obtaining a peptide which is a C-terminal amide derivative according to the process of the present invention, and converting the obtained peptide which is a C-terminal amide derivative to peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate.

In another embodiment, the present invention provides a process for preparing a pharmaceutical formulation comprising combining the peptide acetate obtained according to the processes of the present invention with at least one pharmaceutically acceptable excipient.

### Detailed Description of the Invention

As used herein, the term "ACN" refers to acetonitrile.

As used herein, the term "Boc" refers to t-Butyloxycarbonyl.

As used herein, the term "Bzl" refers to benzyl.

As used herein, the term "Cbz" refers to benzyloxycarbonyl.

As used herein, the term "DCM" refers to dichloromethane.

As used herein, the term "DIEA" refers to diisopropylethylamine.

As used herein, the term "DMF" refers to dimethylformamide.

As used herein, the term "EDT" refers to ethanedithiol.

As used herein, the term "Fmoc" refers to 9-fluorenylmethoxycarbonyl.

As used herein, the term "HBTU" refers to 2-(1H-Benzotriazole-1-yl)-1,1,3,3-tetramethyluronium hexafluorophosphate.

As used herein, the term "HOBt" refers to N-hydroxybenzotriazole.

As used herein, the term "Pbf" refers to pentamethyldihydrobenzofuransulfonyl.

As used herein, the term "SPPS" refers to solid phase peptide synthesis.

As used herein, the term "TBTU" refers to 2-(1H-Benzotriazole-1-yl)-1,1,3,3-tetramethyluronium tetrafluoroborate

As used herein, the term "tBu" refers to tert-butyl.

As used herein, the term "TFA" refers to trifluoroacetic acid.

As used herein, the term "TIS" refers to triisopropylsilane.

As used herein, the term "Trt" refers to trityl.

As used herein, the term "RT" or "room temperature" refers to a temperature of about 18-25°C, preferably about 20-22°C.

As used herein, the term "mild acidic solution" refers to a solution comprising an acid in an inert organic solvent, in a concentration such that during the cleavage of the peptide from the resin the protecting groups remain the peptide.

As used herein, the term "coupling reagent" refers to any product that activates the carboxyl group of the protected peptide fragment.

The invention relates a method for preparing a peptide, which is a C-terminal amide derivative, that comprises a combination of a solid-phase synthesis (SPPS), using a resin as a solid support, to obtain a protected peptide with a carboxylic C-terminus, and a solution-phase synthesis for the amidation of the C-terminus. The invention further relates to protected peptide precursors and to peptide acetates having a purity of at least about 99.0% as determined by HPLC method.

All stages in the process of the present invention are performed under mild conditions, and thus providing a low content of by-products, a high yield and high purity of the final product. In addition, the processes of the present invention require regular commercially available protected amino acids.

The present invention provides a method of preparing a peptide which is a C-terminal amide derivative, comprising: providing amino acid, protected or non-protected, attached in its C-terminal to a super-acid labile resin; coupling said amino acid, with another amino acid, protected or non-protected, in the presence of a coupling reagent; repeating the coupling step to obtain a peptide, wherein the peptide is protected with at least one protecting group which remains on the peptide upon its cleavage from the resin; cleaving said protected peptide from the resin by admixing with a mild acidic solution; and amidating the obtained protected peptide with a suitable amine.

Optionally, the amidation step comprises adding a base. Preferably, the base is diisopropylethylamine.

Preferably, the super-acid labile resin is selected from the group consisting of: chlorotrityl resin, Rink acid resin, NovaSyn TGT resin, and HMPB-AM resin.

Preferably, the coupling reagent is 2-(1H-benzotriazole-1-yl)-1,1,3,3-tetramethyluronium tetrafluoroborate (TBTU).

Preferably, the mild acidic solution is a solution comprising about 0.1 % to about 5% of TFA in an organic inert solvent or a mixture of acetic acid with trifluoroethanol and DCM.

Preferably, prior to the amidation the protected peptide is isolated. Preferably, the isolation is by precipitation, crystallization, extraction, or chromatography. More preferably, the isolation is by precipitation.

Optionally, the peptide obtained after the cleavage from the resin is protected Leuprolide precursor consisting on amino acids having the sequence of: pGlu-His(Trt)-Trp-Ser(tBu)-Tyr(tBu)-D-Leu-Leu-Arg(Pbf)-Pro-OH (SEQ. ID. NO. 1). Preferably, the amidation comprises treating protected Leuprolide precursor with a coupling reagent in the presence of ethyl amine in DMF and diisopropylethylamine, to obtain protected Leuprolide consisting on amino acids having the sequence of: pGlu-His(Trt)-Trp-Ser(tBu)-Tyr(tBu)-D-Leu-Leu-Arg(Pbf)-Pro-NHEt (SEQ. ID. NO. 1). Preferably, after the amidation, the process further comprises: reacting the protected Leuprolide with a an acid composition comprising a TFA solution containing water, TIS and EDT; adding MTBE to obtain a precipitate of Leuprolide consisting on amino acids having the sequence of: pGlu-His-Trp-Ser-Tyr-D-Leu-Leu-Arg-Pro-NHEt (SEQ. ID. NO. 1); and isolating the Leuprolide.

Optionally, the peptide obtained after the cleavage from the resin is protected Goserelin precursor consisting on amino acids having the sequence of: pGlu-His-Trp-Ser-Tyr(Bzl)-D-Ser(tBu)-Leu-Arg(NO2)-Pro- (SEQ. ID. NO. 4). Preferably, the amidation comprises treating protected Goserelin precursor with a coupling reagent in the presence of semicarbazide in DMF/water and diisopropylethylamine, to obtain protected Goserelin consisting on amino acids having the sequence of: pGlu-His-Trp-Ser-Tyr(Bzl)-D-Ser(tBu)-Leu-Arg(NO2)-Pro-HNNHCON2 (SEQ. ID. NO. 4). Preferably, after the amidation, the process further comprises: reacting the protected Goserelin under hydrogenolysis conditions; adding MTBE to obtain a precipitate of Goserelin consisting on amino acids having the sequence of: pGlu-His-Trp-Ser-Tyr-D-Ser(tBu)-Leu-Arg-Pro-HNNHCONH2 (SEQ. ID. NO. 4); and isolating the Goserelin.

Optionally, the peptide obtained after the cleavage from the resin is protected Buserelin precursor consisting on amino acids having the sequence of: pGlu-His-Trp-Ser-Tyr(Bzl)-D-Ser(tBu)-Leu-Arg(NO2)-Pro- (SEQ. ID. NO. 3). Preferably, the amidation comprises treating protected Buserelin precursor with a coupling reagent in the presence of ethyl amine in DMF and diisopropylethylamine, to obtain protected Buserelin consisting on amino acids having the sequence of: pGlu-His-Trp-Ser-Tyr(Bzl)-D-Ser(tBu)-Leu-Arg(NO2)-Pro-NHEt (SEQ. ID. NO. 3). Preferably, after the amidation, the process further comprises: reacting the protected Buserelin under hydrogenolysis conditions; adding MTBE to obtain a precipitate of Buserelin consisting on amino acids having the sequence of: pGlu-His-Trp-Ser-Tyr-D-Ser(tBu)-Leu-Arg-Pro-HNEt (SEQ. ID. NO. 3); and isolating the Buserelin.

Optionally, the peptide obtained after the cleavage from the resin is protected Triptorelin precursor consisting on amino acids having the sequence of: pGlu-His(Trt)-Trp-Ser(tBu)-Tyr(tBu)-D-Trp-Leu-Arg(Pbf)-Pro-Gly-OH (SEQ. ID. NO. 2). Preferably, the amidation comprises treating protected Triptorelin precursor with a coupling reagent in the presence of ammonia in DMF and diisopropylethylamine, to obtain protected Triptorelin consisting on amino acids having the sequence of: pGlu-His(Trt)-Trp-Ser(tBu)-Tyr(tBu)-D-Trp-Leu-Arg(Pbf)-Pro-Gly-NH2 (SEQ. ID. NO. 2). Preferably, after the amidation, the process further comprises: reacting the protected Triptorelin with a an acid composition comprising a TFA solution containing water, TIS and EDT; adding MTBE to obtain a precipitate of Triptorelin consisting on amino acids having the sequence of: pGlu-His-Trp-Ser-Tyr-D-Trp-Leu-Arg-Pro-Gly-NH2 (SEQ. ID. NO. 2); and isolating the Triptorelin.

Optionally, the peptide obtained after the cleavage from the resin is protected Desmopressin precursor consisting on amino acids having the sequence of: Mpa(Trt)-Tyr(tBu)-Phe-Gln(Trt)-Asn-Cys(Acm)-Pro-D-Arg(Pbf)-Gly-OH (SEQ. ID. NO. 5). Preferably, the amidation comprises treating protected Desmopressin precursor with a coupling reagent in the presence of ammonia in DMF and diisopropylethylamine, to obtain protected Desmopressin consisting on amino acids having the sequence of: Mpa(Trt)-Tyr(tBu)-Phe-Gln(Trt)-Asn-Cys(Acm)-Pro-D-Arg(Pbf)-Gly-NH2 (SEQ. ID. NO. 5). Preferably, after the amidation, the process further comprises: reacting the protected Desmopressin with a an acid composition comprising a TFA solution containing water, TIS and EDT; adding ether to obtain a precipitate of non-cyclic Desmopressin consisting on amino acids having the sequence of: Mpa-Tyr-Phe-Gln-Asn-Cys(Acm)-Pro-D-Arg-Gly-NH₂ (SEQ. ID. NO. 5) (non-cyclic Desmopressin); cyclizing the non-cyclic Desmopressin; and isolating Desmopressin having the structure:

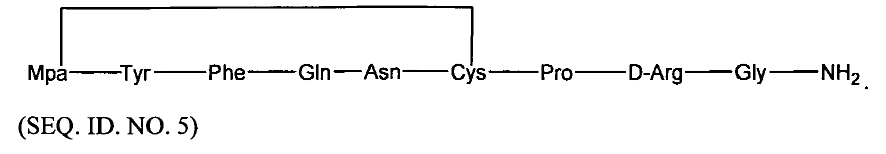

Optionally, the peptide obtained after the cleavage from the resin is protected Lanreotide precursor consisting on amino acids having the sequence of: Boc-D-Nal-Cys(Trt)-Tyr(tBu)-D-Trp-Lys(Boc)-Val-Cys(Acm)-Thr(tBu)-OH (SEQ. ID. NO. 17). Preferably, the amidation comprises treating protected Lanreotide precursor with a coupling reagent in the presence of ammonia in DMF, to obtain protected Lanreotide consisting on amino acids having the sequence of: Boc-D-Nal-Cys(Trt)-Tyr(tBu)-D-Trp-Lys(Boc)-Val-Cys(Acm)-Thr(tBu)-NH₂ (SEQ. ID. NO. 17). Preferably, after the amidation, the process further comprises: reacting the protected Lanreotide with a an acid composition comprising a TFA solution containing water, TIS and EDT; adding ether to obtain a precipitate of non-cyclic Lanreotide consisting on amino acids having the sequence of: D-Nal-Cys-Tyr-D-Trp-Lys-Val-Cys(Acm)-Thr-NH₂ (SEQ. ID. NO. 17); cyclizing the non-cyclic Lanreotide; and isolating Lanreotide consisting on amino acids having the sequence of: D-Nal-Cys-Tyr-D-Trp-Lys-Val-Cys-Thr-NH₂ cyclic (2-7) (SEQ. ID. NO. 17)disulfide.

Optionally, the peptide obtained after the cleavage from the resin is protected Atosiban precursor consisting on amino acids having the sequence of: Mpa(Trt)-D-Tyr(Et)-Ile-Thr(tBu)-Asn(Trt)-Cys(Acm)-Pro-Orn(Boc)-Gly-OH (SEQ. ID. NO. 19). Preferably, the amidation comprises treating protected Atosiban precursor with a coupling reagent in the presence of ammonia in DMF, to obtain protected Atosiban consisting on amino acids having the sequence of: Mpa(Trt)-D-Tyr(Et)-Ile-Thr(tBu)-Asn(Trt)-Cys(Acm)-Pro-Orn(Boc)-Gly-NH₂ (SEQ. ID. NO. 19). Preferably, after the amidation, the process further comprises: reacting the protected Atosiban with a an acid composition comprising a TFA solution containing water, TIS and EDT; adding ether to obtain a precipitate of non-cyclic Atosiban consisting on amino acids having the sequence of: Mpa-D-Tyr(Et)-Ile-Thr-Asn-Cys(Acm)-Pro-Orn-Gly-NH2 (SEQ. ID. NO. 19); cyclizing the non-cyclic Atosiban; and isolating Atosiban consisting on amino acids having the sequence of: Mpa-D-Tyr(Et)-Ile-Thr-Asn-Cys-Pro-Orn-Gly-NH₂, (SEQ. ID. NO. 19) cyclic (1-6) disulfide.

Optionally, the peptide obtained after the cleavage from the resin is protected Exenatide precursor consisting on amino acids having the sequence of: Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEQ. ID. NO. 31). Preferably, the amidation comprises treating protected Exenatide precursor with a coupling reagent in the presence of ammonia in DMF, to obtain protected Exenatide consisting on amino acids having the sequence of: Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-NH₂ (SEQ. ID. NO. 31). Preferably, after the amidation, the process further comprises: reacting the protected Exenatide with a an acid composition comprising a TFA solution containing water, TIS and EDT; adding ether to obtain a precipitate of Exenatide consisting on amino acids having the sequence of: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEQ. ID. NO. 31); and isolating the Exenatide.

Preferably, the isolation of the peptides is by precipitation.

Preferably, the precipitation is from a solvent selected from the group consisting of: methyl-tert-butyl ether (MTBE), diethyl ether, diisopropylether and mixtures thereof. Preferably, the solvent is mixed with methanol, ethanol or acetonitrile.

Preferably, the after the isolation of the peptides the process further comprises: purifying the peptide selected from the group consisting of: Leuprolide, Goserelin, Triptorelin and Eptifibatide by HPLC chromatography, and simultaneously replacing the counter-ion of the peptide with an acetate to obtain peptide acetate; and drying the solution of the peptide acetate selected from the group consisting of: Leuprolide acetate, Goserelin acetate, Triptorelin acetate and Eptifibatide acetate. Preferably, the drying is by: lyophilizing or spray drying.

Preferably, the dried Leuprolide acetate contains less than about 0.1 % D-Ser⁴-Leuprolide.

Preferably, the dried Leuprolide acetate contains less than about 0.2% D-His²-Leuprolide.

Preferably, the dried Leuprolide acetate contains less than about 0.1% D-pGlu¹-Leuprolide

Preferably, the dried Leuprolide acetate contains not more than about 0.1% of any other impurity.

Preferably, the dried Goserelin acetate contains less than about 0.5% of any other impurity.

Preferably, the dried Triptorelin acetate contains less than about 0.5% of any other impurity.

The present invention provides Leuprolide acetate containing less than about 0.1 % D-Ser⁴-Leuprolide.

The present invention provides Leuprolide acetate containing less than about 0.2% D-His²-Leuprolide.

The present invention provides Leuprolide acetate containing less than about 0.1 % D-pGlu¹-Leuprolide

The present invention provides Leuprolide acetate containing not more than about 0.1 % of any other impurity.

The present invention provides Goserelin acetate containing less than about 0.5% of any other impurity.

The present invention provides Triptorelin acetate containing less than about 0.5% of any other impurity.

As another aspect of the present invention relates to protected peptide precursors, the present invention also provides pGlu-His(Trt)-Trp-Ser(tBu)-Tyr(tBu)-D-Leu-Leu-Arg(Pbf)-Pro-OH (SEQ. ID. NO. 1) (protected Leuprolide precursor).

The present invention also provides pGlu-His-Trp-Ser-Tyr(Bzl)-D-Ser(tBu)-Leu-Arg(NO2)-Pro-OH (SEQ. ID. NO. 4) (protected Goserelin precursor).

The present invention also provides pGlu-His(Trt)-Trp-Ser(tBu)-Tyr(tBu)-D-Trp-Leu-Arg(Pbf)-Pro-Gly-OH (SEQ. ID. NO. 2) (protected Triptorelin precursor).

The present invention provides peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate having a purity of at least about 99.0% as determined by HPLC method.

The present invention provides a pharmaceutical composition comprising peptide acetate made by one of the processes of the present invention and at least one pharmaceutically acceptable excipient.

The present invention provides a process for preparing a pharmaceutical formulation comprising combining peptide acetate made by one of the processes of the present invention, with at least one pharmaceutically acceptable excipient.

The present invention provides the use of peptide acetate made by one of the processes of the present invention for the manufacture of a pharmaceutical composition.

The present invention provides a process for preparing peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate comprising obtaining a peptide which is a C-terminal amide derivative according to the process of the present invention, and converting the obtained peptide which is a C-terminal amide derivative to peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate.

The present invention provides a process for preparing a pharmaceutical formulation comprising combining the peptide acetate obtained according to the processes of the present invention with at least one pharmaceutically acceptable excipient.

Methods of administration of a pharmaceutical composition of the present invention can be administered in various preparations depending on the age, sex, and symptoms of the patient. The pharmaceutical compositions can be administered, for example, as tablets, pills, powders, liquids, suspensions, emulsions, granules, capsules, suppositories, injection preparations (solutions and suspensions), and the like.

Pharmaceutical compositions of the present invention can optionally be mixed with peptide acetate obtained in the present invention and other active ingredients. In addition, pharmaceutical compositions of the present invention can contain inactive ingredients such as diluents, carriers, fillers, bulking agents, binders, disintegrants, disintegration inhibitors, absorption accelerators, wetting agents, lubricants, glidants, surface active agents, flavoring agents, and the like.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol and tartaric acid.

Solid and liquid compositions may also be dyed using any pharmaceutically acceptable colorant to improve their appearance and/or facilitate patient identification of the product and unit dosage level.

In liquid pharmaceutical compositions of the present invention, the peptide acetate and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste.

Preservatives and chelating agents such as alcohol, sodium benzoate, butylated hydroxy toluene, butylated hydroxyanisole and ethylenediamine tetraacetic acid may be added at levels safe for ingestion to improve storage stability.

According to the present invention, a liquid composition may also contain a buffer such as guconic acid, lactic acid, citric acid or acetic acid, sodium guconate, sodium lactate, sodium citrate or sodium acetate. Selection of excipients and the amounts used may be readily determined by the formulation scientist based upon experience and consideration of standard procedures and reference works in the field.

When preparing injectable (parenteral) pharmaceutical compositions, solutions and suspensions are sterilized and are preferably made isotonic to blood. Injection preparations may use carriers commonly known in the art. For example, carriers for injectable preparations include, but are not limited to, water, ethyl alcohol, propylene glycol, ethoxylated isostearyl alcohol, polyoxylated isostearyl alcohol, and fatty acid esters of polyoxyethylene sorbitan. One of ordinary skill in the art can easily determine with little or no experimentation the amount of sodium chloride, glucose, or glycerin necessary to make the injectable preparation isotonic. Additional ingredients, such as dissolving agents, buffer agents, and analgesic agents may be added.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and losenges, as well as liquid syrups, suspensions and elixirs.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tabletted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.
The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages can be conveniently presented in unit dosage form and prepared by any of the methods well-known in the pharmaceutical arts.

While the present invention is described with respect to particular examples and preferred embodiments, it is understood that the present invention is not limited to these examples and embodiments. The present invention as claimed therefore includes variations from the particular examples and preferred embodiments described herein, as will be apparent to one of skill in the art.

### Instruments

### HPLC

The following HPLC methods are according to the European Pharmacopia:

### HPLC method for Leuprolide:

**Related substances.** Liquid chromatography (*2.2.29*): use the normalization procedure. *Test solution (a).* Dissolve the substance to be examined in the mobile phase to obtain a concentration of 1.0 mg/ml.
*Test solution (b).* Dilute 1.0 ml of test solution (a) to 20.0 ml with the mobile phase. *Reference solution (a).* Dissolve **leuprorelin** CRS in the mobile phase to obtain a concentration of 1.0 mg/ml.
*Reference solution (b).* Dilute 1.0 ml of reference solution (a) to 20.0 ml with the mobile phase.
*Resolution solution.* Dilute 5.0 ml of reference solution (a) to 50.0 ml with *water R.* To 5 ml of the solution add 100 µl of *1 M sodium hydroxide* and shake vigorously. Heat in an oven at 100 °C for 60 min, cool immediately and add 50 µl of *dilute phosphoric acid R.* Shake vigorously.
*Column:*
- *size: l =* 0.10 m, Ø = 4.6 mm,
- *stationary phase: octadecylsilyl silica gel for chromatography R* (3 µm).
   *Mobile phase:* dissolve about 15.2 g of *triethylamine R* in 800 ml of *water R,* adjust to pH 3.0 with *phosphoric acid R* and dilute to 1000 ml with *water R.* Add 850 ml of this solution to 150 ml of a mixture of 2 volumes of *propanol R* and 3 volumes of *acetonitrile R.*
   *Flow rate*: 1.0-1.5 ml/min.
   *Detection:* spectrophotometer at 220 nm.
   *Injection:* 20 µl of test solution (a) and the resolution solution.
   *Run time:* 90 min.
   *Relative retention* with reference to **leuprorelin** (retention time = 41-49 min):
   impurity E = about 0.7; impurity F = about 0.7; impurity H = about 0.78;
   impurity A = about 0.8; impurity B = about 0.9; impurity I = about 0.94;
   impurity J = about 1.09; impurity C = about 1.2; impurity G = about 1.3;
   impurity K = about 1.31; impurity D = about 1.5.
   *System suitability*: resolution solution:
   - *resolution:* minimum 1.5 between the peaks due to impurity B and **leuprorelin**. *Limits:*
   - *impurity D:* maximum 1.0 per cent,
   - *impurities A, B,* C: for each impurity, maximum 0.5 per cent,
   - *any other impurity*: for each impurity, maximum 0.5 per cent,
   - *total*: maximum 2.5 per cent,
   - *disregard limit:* 0.1 per cent.

### HPLC method for Goserelin

**Related substances**. Liquid chromatography (*2.2.29*).
*Test solution.* Dissolve the substance to be examined in *water R* to obtain a concentration of 1.0 mg/ml.
*Reference solution (a).* Dissolve the contents of a vial of **goserelin** CRS in *water R* to obtain a concentration of 1.0 mg/ml.
*Reference solution (b).* Dilute 1.0 ml of the test solution to 100 ml with *water R. Reference solution (c).* Dilute 1.0 ml of the test solution to 10.0 ml with *water R. Resolution solution (a).* Dissolve the contents of a vial of 4-D-Ser-**goserelin** CRS in *water R* to obtain a concentration of 0.1 mg/ml. Mix equal volumes of this solution and of reference solution (c).
*Resolution solution (b).* Dissolve the contents of a vial of **goserelin** validation mixture CRS with 1.0 ml of *water R.*
*Column:*
- *size:* 1= 0.15 m, Ø = 4.6 mm,
- *stationary phase: octadecylsilyl amorphous organosilica polymer R* (3.5 µm) with a pore size of 12.5 nm,
- *temperature:* 50-55 °C.
*Mobile phase: trifluoroacetic acid R, acetonitrile for chromatography R, water R* (0.5:200:800 *V*/*V*/*V*).
*Flow rate*: 0.7-1.2 ml/min.
*Detection:* spectrophotometer at 220 nm.
*Injection:* 10 µl of the test solution, reference solution (b) and the resolution solutions. *Run time:* 90 min.
*Relative retention* with reference to **goserelin**: impurity A = about 0.67;
impurity C = about 0.78; impurity B = about 0.79; impurity D = about 0.85;
impurity E = about 0.89; impurity F = about 0.92; impurity G = about 0.94;
impurity H = about 0.98; impurity I = about 1.43; impurity J = about 1.53;
impurity K = about 1.67; impurity L = about 1.77.◄
*System suitability*:
- *retention time:* **goserelin** = 40 min to 50 min in the chromatogram obtained with resolution solution (b); adjust the flow rate of the mobile phase if necessary; if adjusting the flow rate does not result in a correct retention time of the principal peak, change the composition of acetonitrile in the mobile phase to obtain the requested retention time for **goserelin;**
- *resolution:* minimum 7.0 between the peaks due to impurity A and **goserelin** in the chromatogram obtained with resolution solution (a);
- *symmetry factor*: 0.8 to 2.5 for the peaks due to impurity A and **goserelin** in the chromatogram obtained with resolution solution (a);
- the chromatogram obtained with resolution solution (b) is similar to the chromatogram supplied with **goserelin** validation mixture CRS. 2 peaks eluting prior to the principal peak and corresponding to impurity E and impurity G, are clearly visible. 3 peaks eluting after the principal peak are clearly visible.
*Limits:*
- *impurity E*: not more than the area of the principal peak in the chromatogram obtained with reference solution (b) (1.0 per cent),
- *any other impurity*: for each impurity, not more than 0.5 times the area of the principal peak in the chromatogram obtained with reference solution (b) (0.5 per cent),◄
- *total*: not more than 2.5 times the area of the principal peak in the chromatogram obtained with reference solution (b) (2.5 per cent),
- *disregard limit:* 0.05 times the area of the principal peak in the chromatogram obtained with reference solution (b) (0.05 per cent).

### Examples

### Example 1: Preparation of pGlu-His(Trt)-Trp-Ser(tBu)-Tyr(tBu)-D-Leu-Leu-Arg(Pbf)-Pro-OH (SEQ. ID. NO. 1) (Leuprolide precursor)

Synthesis of the protected peptide was carried out by a stepwise Fmoc SPPS (solid phase peptide synthesis) procedure starting from the loading of Fmoc-Pro-OH to 2-Cl-Trt-Cl resin. The resin (2-Cl-Trt-Cl resin, 1 kg), after washing, was stirred with a solution of Fmoc-Pro-OH (470 g) in DMF in the presence of diisopropylethylamine for 2 h. After washing of the resin, the Fmoc protecting group was removed by treatment with 20% piperidine in DMF. After washing of residual reagents, the second amino acid (Fmoc-Arg(Pbf)) was introduced to start the first coupling step. The Fmoc protected amino acid was activated in situ using TBTU/HOBt (N-hydroxybenzotriazole) and subsequently coupled to the resin for 50 minutes. Diisopropylethylamine was used during coupling as an organic base. Completion of the coupling was indicated by ninhydrin test. After washing of the resin, the Fmoc protecting group on the α-amine was removed with 20% piperidine in DMF for 20 min. These steps were repeated each time another amino acid was added, according to the peptide sequence. All amino acids used were Fmoc-N^{α} protected except the last amino acid in the sequence, pGlu. Trifunctional amino acids were side chain protected as follows: Ser(t-Bu), Arg(Pbf), Tyr(tBu), and His(Trt). Three equivalents of the activated amino acids were employed in the coupling reactions. At the end of the synthesis, the peptide-resin was washed with DMF, followed by DCM, and dried under vacuum to obtain 2150 g dry peptide-resin.

The peptide, prepared as described above, was cleaved from the resin at RT using a 1% TFA solution in DCM by three repeated washings (15 min each). The acidic peptide solution was neutralized by DIPEA. The solvent was evaporated under reduced pressure and the protected peptide was precipitated by the addition of 10 volumes of water, filtered, and dried in vacuum to obtain 1070 g powder. It was identified by MS as pGlu-His(Trt)-Trp-Ser(tBu)-Tyr(tBu)-D-Leu-Leu-Arg(Pbf)-Pro-OH (SEQ. ID. NO. 1).

### Example 2: Preparation of pGlu-His(Trt)-Trp-Ser(tBu)-Tyr(tBu)-D-Leu-Leu-Arg(Pbf)-Pro-NHEt (SEQ. ID. NO. 1)

pGlu-His(Trt)-Trp-Ser(tBu)-Tyr(tBu)-D-Leu-Leu-Arg(Pbf)-Pro-OH (SEQ. ID. NO. 1) crude peptide (1070 g), prepared as described in Example 1, was reacted with ethyl amine dissolved in DMF. The activation of carboxyl group of the peptide was done in-situ by addition of TBTU/HOBt to the reaction mixture. Diisopropylethyl amine was used as organic base. Completion of the reaction was monitored by HPLC analysis. At the end of the reaction, the DMF solution was added slowly to water and crude protected peptide was precipitated as an off-white solid. The peptide was separated by filtration, dried, and identified by MS as pGlu-His(Trt)-Trp-Ser(tBu)-Tyr(tBu)-D-Leu-Leu-Arg(Pbf)-Pro-NHEt (SEQ. ID. NO. 1).

### Example 3: Preparation of pGlu-His-Trp-Ser-Tyr-D-Leu-Leu-Arg-Pro-NHEt (SEQ. ID. NO. 1)

The protecting groups from pGlu-His(Trt)-Trp-Ser(tBu)-Tyr(tBu)-D-Leu-Leu-Arg(Pbf)-Pro-NHEt (SEQ. ID. NO. 1) (about 1000 g obtained in Example 2) were removed using a 95% TFA, 2.5% TIS, 2.5% EDT solution for 2 hours at room temperature. The product was precipitated by the addition of 10 volumes of MTBE, filtered, and dried in vacuum to obtain 680 g product.

The crude peptide was dissolved in an aqueous solution of acetonitrile. The resulting solution was loaded on a C₁₈ RP-HPLC column and purified to obtain fractions containing Leuprolide salt at a purity of >99.0%. After treatment on RP-HPLC to replace the counter-ion with acetate, the fractions containing the peptide were collected and lyophilized to obtain final dry peptide (286 g, 42% yield), >99.0% pure (HPLC). The peptide contained less than 0.1% D-Ser⁴-Leuprolide, less than 0.4% D-His²-Leuprolide, less than 0.1% D-pGlu¹-Leuprolide, and not more than 0.1% of any other impurity.

In another preparation similar to Example 1, treated in a similar way as described above, the peptide was obtained >99.5% pure, contained less than 0.1% D-Ser⁴-Leuprolide, less than 0.2% D-His²-Leuprolide, less than 0.1% D-pGlu¹-Leuprolide, and not more than 0.1 % of any other impurity.

### Example 4: Preparation of pGlu-His-Trp-Ser-Tyl(Bzl)-D-Ser(tBu)-Leu-Arg(NO2)-Pro-OH (SEQ. ID. NO. 4) (Goserelin precursor)

Synthesis of the protected peptide was carried out by a stepwise Fmoc SPPS (solid phase peptide synthesis) procedure starting from the loading of Fmoc-Pro-OH to 2-Cl-Trt-Cl resin. The resin (2-Cl-Trt-Cl resin, 100 g), after washing, was stirred with a solution of Fmoc-Pro-OH in DMF in the presence of diisopropylethylamine for 2 h. After washing of the resin, the Fmoc protecting group was removed by treatment with 20% piperidine in DMF. After washing of residual reagents, the second amino acid (Fmoc-Arg(NO₂)) was introduced to start the first coupling step. The Fmoc protected amino acid was activated in situ using TBTU/HOBt (N-hydroxybenzotriazole) and subsequently coupled to the resin for 50 minutes. Diisopropylethylamine was used during coupling as an organic base. Completion of the coupling was indicated by Ninhydrin test. After washing of the resin, the Fmoc protecting group on the α-amine was removed with 20% piperidine in DMF for 20 min. These steps were repeated each time with another amino acid, according to the peptide sequence. All amino acids used were Fmoc-N^{α} protected, except the last amino acid in the sequence, pGlu. Trifunctional amino acids were side chain protected as follows: D-Ser(t-Bu), Arg(NO₂), Tyr(Bzl), and His(Fmoc). Three equivalents of the activated amino acids were employed in the coupling reactions. At the end of the synthesis, the peptide-resin was washed with DMF, followed by DCM, and dried under vacuum to obtain 210 g dry peptide-resin.

The peptide, prepared as described above, was cleaved from the resin at RT using a 1% TFA solution in DCM by three repeated washings (15 min each). The acidic peptide solution was neutralized by DIPEA. The solvent was evaporated under reduced pressure and the protected peptide was precipitated by the addition of 10 volumes of water, filtered, and dried in vacuum to obtain 98 g powder. The peptide was identified by MS as pGlu-His-Trp-Ser-Tyr(Bzl)-D-Ser(tBu)-Leu-Arg(NO2)-Pro-OH (SEQ. ID. NO. 4).

### Example 5: Preparation of pGlu-His-Trp-Ser-Tyr(Bzl)-D-Ser(tBu)-Leu-Arg(NO2)-Pro-HNNHCONH2 (SEQ. ID. NO. 4)

pGlu-His-Trp-Ser-Tyr(Bzl)-D-Ser(tBu)-Leu-Arg(NO2)-Pro-OH (SEQ. ID. NO. 4) crude peptide (98 g), prepared as described in Example 4, was reacted with semicarbazide hydrochloride dissolved in DMF/water. The activation of the carboxyl group of the peptide was done in-situ by addition of TBTU/HOBt to the reaction mixture. Diisopropylethyl amine was used as an organic base. Completion of the reaction was monitored by HPLC analysis. At the end of the reaction, the DMF solution was added slowly to water and crude protected peptide was precipitated as an off-white solid. The peptide was separated by filtration, dried, and identified by MS as pGlu-His-Trp-Ser-Tyr(Bzl)-D-Ser(tBu)-Leu-Arg(NO2)-Pro-HNNHCONH2 (SEQ. ID. NO. 4).

### Example 6: Preparation of pGlu-His-Trp-Ser-Tyr-D-Ser(tBu)-Leu-Arg-Pro-HNNHCONH2 (SEQ. ID. NO. 4)

The protecting groups (except for the tBu on the D-Ser) from pGlu-His-Trp-Ser-Tyr(Bzl)-D-Ser(tBu)-Leu-Arg(NO2)-Pro-HNNHCONH2 (SEQ. ID. NO. 4) (102 g) obtained in Example 5 were removed by hydrogenolysis on 5% Pd/C in DMF. The product was precipitated by the addition of 10 volumes of MTBE, filtered and dried in vacuum to obtain 91 g product.

The crude peptide was dissolved in an acetonitrile aqueous solution. The resulting solution was loaded on a C₁₈ RP-HPLC column and purified to obtain fractions containing Goserelin salt at a purity of >99.0%. After treatment on RP-HPLC to replace the counter-ion with acetate, the peptide-containing fractions were collected and lyophilized to obtain final dry peptide (32 g), >99.0% pure, total impurities less than 1.0%, and each impurity less than 0.5%.

### Example 7: Preparation of pGlu-His-Trp-Ser-Tyr(Bzl)-D-Ser(tBu)-Leu-Arg(NO2)-Pro-NHEt (SEQ. ID. NO. 3) (Buserelin precursor)

pGlu-His-Trp-Ser-Tyr(Bzl)-D-Ser(tBu)-Leu-Arg(NO2)-Pro-OH (SEQ. ID. NO. 3) crude peptide (100 g), prepared as described in Example 4, was reacted with ethyl amine dissolved in DMF. The activation of the carboxyl group of the peptide was done in-situ by addition of TBTU/HOBt to the reaction mixture. Diisopropylethyl amine was used as an organic base. Completion of the reaction was monitored by HPLC analysis. At the end of the reaction, the DMF solution was added slowly to water and crude protected peptide was precipitated as an off-white solid. It was separated by filtration, dried, and identified by MS as pGlu-His-Trp-Ser-Tyr(Bzl)-D-Ser(tBu)-Leu-Arg(NO2)-Pro-NHEt (SEQ. ID. NO. 3).

### Example 8: Preparation of pGlu-His-Trp-Ser-Tyr-D-Ser(tBu)-Leu-Arg-Pro-HNEt (SEQ. ID. NO. 3)

The protecting groups (except for the tBu on the D-Ser) from pGlu-His-Trp-Ser-Tyr(Bzl)-D-Ser(tBu)-Leu-Arg(NO2)-Pro-NHEt (SEQ. ID. NO. 3) (98 g), obtained in Example 7, were removed by hydrogenolysis on Pd/C 5% in DMF. The product was precipitated by the addition of 10 volumes of MTBE, filtered, and dried in vacuum to obtain 84 g product.

The crude peptide was dissolved in an aqueous solution of acetonitrile. The resulting solution was loaded on a C₁₈ RP-HPLC column and purified to obtain fractions containing Buserelin salt at a purity of >99.0%. After treatment on RP-HPLC to replace the counter-ion with acetate, the fractions were collected and lyophilized to obtain final dry peptide (28 g), >99.0% pure, total impurities less than 1.0% and each impurity less than 0.5%.

### Example 9: Preparation of pGlu-His(Trt)-Trp-Ser(tBu)-Tyr(tBu)-D-Trp-Leu-Arg(Pbf)-Pro-Gly-OH (SEQ. ID. NO. 2)(Triptorelin precursor)

Synthesis of the protected peptide was carried out by a stepwise Fmoc SPPS (solid phase peptide synthesis) procedure starting from loading of Fmoc-Gly-OH to 2-Cl-Trt-Cl resin. The resin (2-Cl-Trt-Cl resin, 100 g), after washing, was stirred with a solution of Fmoc-Gly-OH in DMF in the presence of diisopropylethylamine for 2 h. After washing of the resin, the Fmoc protecting group was removed by treatment with 20% piperidine in DMF. After washing of residual reagents, the second amino acid (Fmoc-Pro-OH) was introduced to start the first coupling step. The Fmoc protected amino acid was activated in situ using TBTU/HOBt (N-hydroxybenzotriazole) and subsequently coupled to the resin for 50 minutes. Diisopropylethylamine was used during coupling as an organic base. Completion of the coupling was indicated by ninhydrin test. After washing of the resin, the Fmoc protecting group on the α-amine was removed with 20% piperidine in DMF for 20 min. These steps were repeated each time with another amino acid according to the peptide sequence. All amino acids used were Fmoc-N^{α} protected except the last amino acid in the sequence, pGlu. Trifunctional amino acids were side chain protected as follows: Ser(tBu), Arg(Pbf), Tyr(tBu), and His(Trt). Three equivalents of the activated amino acids were employed in the coupling reactions. At the end of the synthesis, the peptide-resin was washed with DMF, followed by DCM, and dried under vacuum to obtain 236 g dry peptide-resin.

The peptide, prepared as described above, was cleaved from the resin at RT using a 1% TFA solution in DCM by three repeated washings (15 min each). The acidic peptide solution was neutralized by DIPEA. The solvent was evaporated under reduced pressure and the protected peptide was precipitated by the addition of 10 volumes of water, filtered, and dried in vacuum to obtain 133 g powder. The peptide was identified by MS as pGlu-His(Trt)-Trp-Ser(tBu)-Tyr(tBu)-D-Trp-Leu-Arg(Pbf)-Pro-Gly-OH (SEQ. ID. NO. 2).

### Example 10: Preparation of pGlu-His(Trt)-Trp-Ser(tBu)-Tyl(tBu)-D-Trp-Leu-Arg(Pbf)-Pro-Gly-NH2 (SEQ. ID. NO. 2)

pGlu-His(Trt)-Trp-Ser(tBu)-Tyr(tBu)-D-Trp-Leu-Arg(Pbf)-Pro-Gly-OH (SEQ. ID. NO. 2) crude peptide (prepared as described in Example 9) was reacted with ammonia dissolved in DMF. The activation of the carboxyl group of the peptide was done in-situ by addition of TBTU/HOBt to the reaction mixture. Diisopropylethyl amine was used as an organic base. Completion of the reaction was monitored by HPLC analysis. At the end of the reaction, the DMF solution was added slowly to water and crude protected peptide was precipitated as an off-white solid. The peptide was separated by filtration, dried, and identified by MS as pGlu-His(Trt)-Trp-Ser(tBu)-Tyr(tBu)-D-Trp-Leu-Arg(Pbf)-Pro-Gly-NH2 (SEQ. ID. NO. 2).

### Example 11: Preparation of pGlu-His-Trp-Ser-Tyr-D-Trp-Leu-Arg-Pro-Gly-NH2 (SEQ. ID. NO. 2)

The protecting groups from pGlu-His(Trt)-Trp-Ser(tBu)-Tyr(tBu)-D-Trp-Leu-Arg(Pbf)-Pro-Gly-NH2 (SEQ. ID. NO. 2)(122 g), obtained in Example 10, were removed using a 95% TFA, 2.5% TIS, 2.5% EDT solution for 2 hours at room temperature. The product was precipitated by the addition of 10 volumes of MTBE, filtered, and dried in vacuum to obtain 85 g product.

The crude peptide was dissolved in an aqueous solution of acetonitrile. The resulting solution was loaded on a C₁₈ RP-HPLC column and purified to obtain fractions containing Triptorelin salt at a purity of >99.0%. After treatment on RP-HPLC to replace the counter-ion with acetate, the fractions were collected and lyophilized to obtain final dry Triptorelin acetate (27 g), >99.0% pure, total impurities less than 1.0% and each impurity less than 0.5%.

By a similar procedure, Triptorelin solution was loaded on a RP-C 18 resin and its counter-ion was replaced with pamoate. The resulting solution was lyophilized to obtain Triptorelin pamoate, >99.0% pure, total impurities less than 1.0%, and each impurity less than 0.5%.

### Example 12: Preparation of Mpa(Trt)-Tyr(tBu)-Phe-Gln(Trt)-Asn-Cys(Acm)-Pro-D-Arg(Pbf)-Gly-OH (SEQ. ID. NO. 5) (Desmopressin precursor)

Synthesis of the peptide is carried out by a regular stepwise Fmoc SPPS procedure starting from 2-Cl-Trt-chloride resin. The first amino acid (Fmoc-Gly) is loaded on the resin as described in previous examples to obtain a substitution of about 0.7 mmol/g of amino acid/resin. After washing of the resin and removal of the Fmoc group by treatment with piperidine/DMF, the second amino acid, Fmoc-D-Arg(Pbf), is introduced to continue sequence elongation. Fmoc protected amino acids are activated in situ using TBTU/HOBt and subsequently coupled to the resin over about 50 minutes. Diisopropylethylamine or collidine is used during coupling as an organic base. Completion of the coupling is indicated by ninhydrin test. After washing of the resin, the Fmoc protecting group on the α-amine is removed with 20% piperidine in DMF for 20 min. These steps are repeated each time with another amino acid according to the peptide sequence. All amino acids used are Fmoc-N^{α} protected except the last building block in the sequence, Trt-Mpa. Trifunctional amino acids are side chain protected as follows: Gln(Trt), D-Arg(Pbf), Tyr(tBu), and Cys(Acm). Three equivalents of the activated amino acids are employed in the coupling reactions. At the end of the synthesis, the peptide-resin is washed with DMF, followed by DCM, and dried under vacuum to obtain dry peptide-resin.

The peptide, prepared as described above, is cleaved from the resin at RT using a 1% TFA in DCM solution by three repeated washings (15 min each). The acidic peptide solution is neutralized by DIPEA. The product is precipitated by the addition of 10 volumes of water, filtered, and dried in vacuum to obtain dry powder. The peptide is identified by LC/MS as Mpa(Trt)-Tyr(tBu)-Phe-Gln(Trt)-Asn-Cys(Acm)-Pro-D-Arg(Pbf)-Gly-OH (SEQ. ID. NO. 5).

### Example 13: Preparation of Mpa-Tyr-Phe-Gln-Asn-Cys(Acm)-Pro-D-Arg-Gly-NH₂ (SEQ. ID. NO. 5)

Mpa(Trt)-Tyr(tBu)-Phe-Gln(Trt)-Asn-Cys(Acm)-Pro-D-Arg(Pbf)-Gly-OH (SEQ. ID. NO. 5) crude peptide (prepared as described in Example 12) is reacted with ammonia dissolved in DMF. The activation of carboxyl group of the peptide is done in-situ by addition of TBTU/HOBt to the reaction mixture. Diisopropylethyl amine is used as an organic base. Completion of the reaction is monitored by HPLC analysis. At the end of reaction the DMF solution is added slowly to water and crude protected peptide is precipitated as off-white solid. The peptide is separated by filtration, dried, and identified by MS as Mpa(Trt)-Tyr(tBu)-Phe-Gln(Trt)-Asn-Cys(Acm)-Pro-D-Arg(Pbf)-Gly-NH2 (SEQ. ID. NO. 5).

The protected peptide above is treated with a cocktail containing 95% TFA, 2.5% TIS, 2.5% EDT for 2 hours at room temperature. The product is precipitated by the addition of 10 volumes of ether, filtered, and dried in vacuum to obtain crude product (Mpa-Tyr-Phe-Gln-Asn-Cys(Acm)-Pro-D-Arg-Gly-NH₂) (SEQ. ID. NO 5).

### Example 14: Preparation of Desmopressin

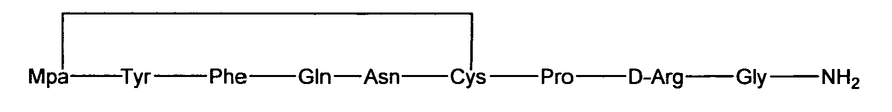

Mpa-Tyr-Phe-Gln-Asn-Cys(Acm)-Pro-D-Arg-Gly-NH2 (SEQ. ID. NO. 5) crude peptide (prepared as described in Example 13) is purified on a preparative C₁₈ RP-HPLC column. Fractions containing >95% pure product are combined and diluted to a concentration of about 1 g/L. An equimolar amount of iodine in acetic acid is added under vigorous mixing at room temperature and subsequently excess iodine is neutralized by a small amount of ascorbic acid. The resulting solution is loaded on a C₁₈ RP-HPLC column and purified to obtain fractions containing desmopressin salt at a purity of >98.5%. After exchange of the counter-ion with acetate (on RP-HPLC), the fractions are collected and lyophilized to obtain final dry peptide, 14.9 g (>99.0% pure).

### Example 15: Preparation of Boc-D-Ala-D-2-Nal-Ala-Trp-D-Phe-Lys(Boc)-OH

Synthesis of the peptide is carried out by a regular stepwise Fmoc SPPS procedure starting from 2-Cl-Trt-chloride resin. The first amino acid, Fmoc-Lys(Boc)-OH, is loaded on the resin as described in previous examples to obtain a loading of about 0.7 mmol/g of amino acid/resin. After washing of the resin and removal of the Fmoc group by treatment with piperidine/DMF, the second amino acid (Fmoc-D-Phe-OH) is introduced to continue sequence elongation. Fmoc protected amino acids are activated in situ using TBTU/HOBt and subsequently coupled to the resin over about 50 minutes. Diisopropylethylamine or collidine is used during coupling as an organic base. Completion of the coupling is indicated by ninhydrin test. After washing of the resin, the Fmoc protecting group on the α-amine is removed with 20% piperidine in DMF for 20 min. These steps are repeated each time with another amino acid according to the peptide sequence. All amino acids used, except the last amino acid (Boc-D-Ala-OH), are Fmoc-N^{α} protected. Trifunctional amino acids are side chain protected as follows: Lys(Boc). Three equivalents of the activated amino acids are used in the coupling reactions. At the end of the synthesis, the peptide-resin is washed with DMF, followed by DCM, and dried under vacuum to obtain dry peptide-resin.

The peptide, prepared as described above, is cleaved from the resin at RT using a 1% TFA in DCM solution by three repeated washings (15 min each). The acidic peptide solution is neutralized by DIPEA. The product is precipitated by the addition of 10 volumes of water, filtered, and dried in vacuum to obtain crude peptide powder.

### Example 16: Boc-D-Ala-D-2-Nal-Ala-Trp-D-Phe-Lys(Boc)-NH₂(SEQ. ID. NO. 13)

Crude protected peptide (prepared as described in Example 31) is reacted with ammonia dissolved in DMF. The activation of the carboxyl group of the peptide is done in-situ by addition of TBTU/HOBt to the reaction mixture. Diisopropylethyl amine is used as an organic base. Completion of the reaction is monitored by HPLC analysis. At the end of the reaction, the DMF solution is added slowly to water and crude protected peptide is precipitated as an off-white solid. The peptide is separated by filtration and dried.

### Example 17: D-Ala-D-2-Nal-Ala-Trp-D-Phe-Lys-NH2 (SEQ. ID. NO. 13)

The protected peptide from Example 32 is treated with a cocktail containing 95% TFA, 5% water for 2 hours at room temperature. The product is precipitated by the addition of 10 volumes of ether, filtered, and dried in vacuum to obtain crude peptide, D-Ala-D-2-Nal-Ala-Trp-D-Phe-Lys-NH2 (SEQ. ID. NO. 13).

The crude peptide is purified on a preparative C₁₈ RP-HPLC column. Fractions containing >98.5% pure product are combined and reloaded on a RP-HPLC column for ion exchange with acetate. After exchange of the counterion, the fractions are collected and lyophilized to obtain final dry peptide, >99.0% pure.

### Example 18: Ac-D-Nal-D-Cpa-D-Pal-Ser-Tyr-D-Cit-Leu-Arg-Pro-D-Ala-NH2 (SEQ. ID. NO. 15)

The protected peptide above is treated with a cocktail containing 95% TFA, 2.5% TIS, 2.5% EDT for 2 hours at room temperature. The product is precipitated by the addition of 10 volumes of ether, filtered, and dried in vacuum to obtain crude peptide, Ac-D-Nal-D-Cpa-D-Pal-Ser-Tyr-D-Cit-Leu-Arg-Pro-D-Ala-NH2 (SEQ. ID. NO. 15).

The crude peptide is purified on a preparative C₁₈ RP-HPLC column. Fractions containing >98.5% pure product are combined and reloaded on a RP-HPLC column for ion exchange with acetate. After exchange of the counterion, the fractions are collected and lyophilized to obtain final dry peptide >99.0% pure.

### Example 19: Preparation of Boc-D-Nal-Cys(Trt)-Tyr(tBu)-D-Trp-Lys(Boc)-Val-Cys(Acm)-Thr(tBu)-OH (SEQ. ID. NO. 17)(Lanreotide precursor)

Synthesis of the peptide is carried out by a regular stepwise Fmoc SPPS procedure starting from 2-Cl-Trt-chloride resin. The first amino acid (Fmoc-Thr(tBu)) is loaded on the resin as described in previous examples to obtain a loading of about 0.7 mmol/g of amino acid/resin. After washing of the resin and removal of the Fmoc group by treatment with piperidine/DMF, the second amino acid (Fmoc-Cys(Acm)) is introduced to continue sequence elongation. Fmoc protected amino acids are activated in situ using TBTU/HOBt and subsequently coupled to the resin over about 50 minutes. Diisopropylethylamine or collidine is used during coupling as an organic base. Completion of the coupling is indicated by ninhydrin test. After washing of the resin, the Fmoc protecting group on the α-amine is removed with 20% piperidine in DMF for 20 min. These steps are repeated each time with another amino acid according to the peptide sequence. All amino acids used are Fmoc-N^{α} protected. Trifunctional amino acids are side chain protected as follows: Cys(Trt) and Cys(Acm), Tyr(tBu), Thr(tBu), and Lys(Boc). Three equivalents of the activated amino acids are used in the coupling reactions. At the end of the synthesis, the peptide-resin is washed with DMF, followed by DCM, and dried under vacuum to obtain dry peptide-resin.

The peptide, prepared as described above, is cleaved from the resin at RT using a 1% TFA in DCM solution by three repeated washings (15 min each). The acidic peptide solution is neutralized by DIPEA. The product is precipitated by the addition of 10 volumes of water, filtered, and dried in vacuum to obtain crude peptide powder.

### Example 20: Preparation of H-D-Nal-Cys-Tyr-D-Trp-Lys-Val-Cys(Acm)-Thr-NH₂ (SEQ. ID. NO. 17)

Crude peptide (prepared as described in Example 43) is reacted with ammonia dissolved in DMF. The activation of the carboxyl group of the peptide is done in-situ by addition of TBTU/HOBt to the reaction mixture. Diisopropylethyl amine is used as an organic base. Completion of the reaction is monitored by HPLC analysis. At the end of the reaction, the DMF solution is added slowly to water and crude protected peptide is precipitated as an off-white solid. It is separated by filtration and dried. The protected peptide above is treated with a cocktail containing 95% TFA, 2.5% TIS, 2.5% EDT for 2 hours at room temperature. The product is precipitated by the addition of 10 volumes of ether, filtered, and dried in vacuum to obtain crude semi-protected peptide, D-Nal-Cys-Tyr-D-Trp-Lys-Val-Cys(Acm)-Thr-NH₂ (SEQ. ID. NO. 17).

### Example 21: Preparation of Lanreotide

D-Nal-Cys-Tyr-D-Trp-Lys-Val-Cys(Acm)-Thr-NH₂ (SEQ. ID. NO. 17) crude peptide (prepared as described in Example 44) is purified on a preparative C₁₈ RP-HPLC column. Fractions containing >95% pure product are combined and diluted to a concentration of about 1 g/L. An equimolar amount of iodine in acetic acid is added under vigorous mixing at room temperature and subsequently excess iodine is neutralized by a small amount of ascorbic acid. The resulting solution is loaded on a C₁₈ RP-HPLC column and purified to obtain fractions containing Lanreotide trifluoroacetate at a purity of >98.5%. After exchange of the counterion with acetate (on RP-HPLC), the fractions are collected and lyophilized to obtain final dry peptide, >99.0% pure.

### Example 22: Preparation of Mpa(Trt)-D-Tyr(Et)-Ile-Thr(tBu)-Asn(Trt)-Cys(Acm)-Pro-Orn(Boc)-Gly-OH (SEQ. ID. NO. 19) (Atosiban precursor)

Synthesis of the peptide is carried out by a regular stepwise Fmoc SPPS procedure starting from 2-Cl-Trt-chloride resin. The first amino acid (Fmoc-Gly) is loaded on the resin as described in previous examples to obtain a loading of about 0.7 mmol/g of amino acid/resin. After washing of the resin and removal of the Fmoc group by treatment with piperidine/DMF, the second amino acid (Fmoc-Orn(Boc)) is introduced to continue sequence elongation. Fmoc protected amino acids are activated in situ using TBTU/HOBt and subsequently coupled to the resin over about 50 minutes. Diisopropylethylamine or collidine is used during coupling as an organic base. Completion of the coupling is indicated by ninhydrin test. After washing of the resin, the Fmoc protecting group on the α-amine is removed with 20% piperidine in DMF for 20 min. These steps are repeated each time with another amino acid according to the peptide sequence. All amino acids used are Fmoc-N^{α} protected. Trifunctional amino acids are side chain protected as follows: Cys(Acm), Thr(tBu), Asn(Trt), and Orn(Boc). Three equivalents of the activated amino acids are used in the coupling reactions. At the end of the synthesis, the peptide-resin is washed with DMF, followed by DCM, and dried under vacuum to obtain dry peptide-resin.

The peptide, prepared as described above, is cleaved from the resin at RT using a 1% TFA in DCM solution by three repeated washings (15 min each). The acidic peptide solution is neutralized by DIPEA. The product is precipitated by the addition of 10 volumes of water, filtered, and dried in vacuum to obtain crude peptide powder.

### Example 23: Mpa-D-Tyr(Et)-Ile-Thr-Asn-Cys(Acm)-Pro-Orn-Gly-NH₂ (SEQ. ID. NO. 19)

Crude peptide (prepared as described in Example 49) is reacted with ammonia dissolved in DMF. The activation of the carboxyl group of the peptide is done in-situ by addition of TBTU/HOBt to the reaction mixture. Diisopropylethyl amine is used as an organic base. Completion of the reaction is monitored by HPLC analysis. At the end of the reaction, the DMF solution is added slowly to water and crude protected peptide is precipitated as an off-white solid. The peptide is separated by filtration and dried.

The protected peptide above is treated with cocktail containing 95% TFA, 2.5% TIS, 2.5% EDT for 2 hours at room temperature. The product is precipitated by the addition of 10 volumes of ether, filtered, and dried in vacuum to obtain crude semi-protected peptide, Mpa-D-Tyr(Et)-Ile-Thr-Asn-Cys(Acm)-Pro-Orn-Gly-NH₂ (SEQ. ID. NO. 19).

### Example 24: Preparation of Atosiban

Mpa-D-Tyr(Et)-Ile-Thr-Asn-Cys(Acm)-Pro-Orn-Gly-NH₂ (SEQ. ID. NO. 19) crude peptide (prepared as described in Example 50) is purified on a preparative C₁₈ RP-HPLC column. Fractions containing >95% pure product are combined and diluted to a concentration of about 1 g/L. An equimolar amount of iodine in acetic acid is added under vigorous mixing at room temperature and subsequently excess iodine is neutralized by a small amount of ascorbic acid. The resulting solution is loaded on a C₁₈ RP-HPLC column and purified to obtain fractions containing Atosiban trifluoroacetate at a purity of >98.5%. After exchange of the counterion with acetate (on RP-HPLC), the fractions are collected and lyophilized to obtain final dry peptide, >99.0% pure.

### Example 25: Preparation of Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEQ. ID. NO. 31) (Exenatide precursor)

Synthesis of the peptide is carried out by a regular stepwise Fmoc SPPS procedure starting from 2-Cl-Trt-chloride resin. The first amino acid (Fmoc-Ser(tBu)) is loaded on the resin as described in previous examples to obtain a loading of about 0.7 mmol/g of amino acid/resin. After washing of the resin and removal of the Fmoc group by treatment with piperidine/DMF, the second amino acid (Fmoc-Pro) is introduced to continue sequence elongation. Fmoc protected amino acids are activated in situ using TBTU/HOBt and subsequently coupled to the resin over about 50 minutes. Diisopropylethylamine or collidine is used during coupling as an organic base. Completion of the coupling is indicated by ninhydrin test. After washing of the resin, the Fmoc protecting group on the □-amine is removed with 20% piperidine in DMF for 20 min. These steps are repeated each time with another amino acid according to the peptide sequence. All amino acids used are Fmoc-N□ protected except the last amino acid, which is protected with Boc. Trifunctional amino acids are side chain protected as follows: Ser(tBu), Thr(tBu), Glu(tBu), Gln(Trt), His(Trt), Arg(Pbf), and Asp(tBu). Three equivalents of the activated amino acids are used in the coupling reactions. At the end of the synthesis, the peptide-resin is washed with DMF, followed by DCM, and dried under vacuum to obtain dry peptide-resin.

The peptide, prepared as described above, is cleaved from the resin at RT using a 1% TFA in DCM solution by three repeated washings (15 min each). The acidic peptide solution is neutralized by DIPEA. The product is precipitated by the addition of 10 volumes of water, filtered and dried in vacuum to obtain crude peptide powder.

### Example 26: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEQ. ID. NO. 31) (Exenatide)

Crude peptide (prepared as described in Example 84) is reacted with ammonia dissolved in DMF. The activation of the carboxyl group of the peptide is done in-situ by addition of TBTU/HOBt to the reaction mixture. Diisopropylethyl amine is used as an organic base. Completion of the reaction is monitored by HPLC analysis. At the end of the reaction, the DMF solution is added slowly to water and crude protected peptide is precipitated as an off-white solid. The peptide is separated by filtration and dried.

The protected peptide above is treated with a cocktail containing 95% TFA, 2.5% TIS, 2.5% EDT for 2 hours at room temperature. The product is precipitated by the addition of 10 volumes of ether, filtered, and dried in vacuum to obtain crude peptide. Crude peptide is purified on a preparative C18 RP-HPLC column to obtain fractions containing peptide solution at a purity of >98.5%. After exchange of the counterion with acetate (on RP-HPLC), the fractions are collected and lyophilized to obtain final dry peptide, >99.0% pure.

Further aspects and features of the present invention are set out in the following numbered clauses.
1. A method of preparing a peptide which is a C-terminal amide derivative, comprising:
   a) providing amino acid, protected or non-protected, attached in its C-terminal to a super-acid labile resin;
   b) coupling said amino acid, with another amino acid, protected or non-protected, in the presence of a coupling reagent;
   c) repeating step b) to obtain a peptide, wherein the peptide is protected with at least one protecting group which remains on the peptide upon its cleavage from the resin;
   d) cleaving said protected peptide from the resin by admixing with a mild acidic solution; and
   e) amidating the protected peptide obtained in step d) with a suitable amine.
2. The process of clause 1, wherein the amidation in step e) is in the presence of a base.
3. The process of clause 2, wherein the base is diisopropylethylamine.
4. The process of clause 1, wherein the super-acid labile resin is selected from the group consisting of: chlorotrityl resin, Rink acid resin, NovaSyn TGT resin, and HMPB-AM resin.
5. The process of clause 1 wherein the coupling reagent is 2-(1H-benzotriazole-1-yl)-1,1,3,3-tetramethyluronium tetrafluoroborate (TBTU).
6. The process of clause 1, wherein the mild acidic solution in step d) is a solution comprising about 0.1% to about 5% of TFA in an organic inert solvent or a mixture of acetic acid with trifluoroethanol and DCM.
7. The process of clause 1, wherein the protected peptide with the resin obtained in step d) is isolated prior to step e).
8. The process of clause 7, wherein the isolation is by precipitation, crystallization, extraction, or chromatography.
9. The process of clause 8, wherein the isolation is by precipitation.
10. The process of clause 1, wherein the protected peptide obtained in step d) is protected Leuprolide precursor consisting on amino acids having the sequence of: pGlu-His(Trt)-Trp-Ser(tBu)-Tyr(tBu)-D-Leu-Leu-Arg(Pbf)-Pro-OH (SEQ. ID. NO. 1).
11. Protected Leuprolide precursor consisting on amino acids having the sequence of: pGlu-His(Trt)-Trp-Ser(tBu)-Tyr(tBu)-D-Leu-Leu-Arg(Pbf)-Pro-OH (SEQ. ID. NO. 1).
12. The process of clause 10, wherein the amidation in step e) comprises treating the protected peptide, obtained in step d), with a coupling reagent in the presence of ethyl amine in DMF and diisopropylethylamine, to obtain protected Leuprolide consisting on amino acids having the sequence of: pGlu-His(Trt)-Trp-Ser(tBu)-Tyr(tBu)-D-Leu-Leu-Arg(Pbf)-Pro-NHEt (SEQ. ID. NO. 1).
13. The process of clause 12, further comprising:
   a) reacting the protected Leuprolide with a an acid composition comprising a TFA solution containing water, TIS and EDT;
   b) adding MTBE to obtain a precipitate of Leuprolide consisting on amino acids having the sequence of: pGlu-His-Trp-Ser-Tyr-D-Leu-Leu-Arg-Pro-NHEt (SEQ. ID. NO. 1); and
   c) isolating the Leuprolide.
14. The process of clause 1, wherein the protected peptide obtained in step d) is protected Goserelin precursor consisting on amino acids having the sequence of: pGlu-His-Trp-Ser-Tyr(Bzl)-D-Ser(tBu)-Leu-Arg(NO2)-Pro- (SEQ. ID. NO. 4).
15. Protected Goserelin precursor consisting on amino acids having the sequence of: pGlu-His-Trp-Ser-Tyr(Bzl)-D-Ser(tBu)-Leu-Arg(NO2)-Pro- (SEQ. ID. NO. 4).
16. The process of clause 14, wherein the amidation in step e) comprises treating the protected peptide, obtained in step d), with a coupling reagent in the presence of semicarbazide in DMF/water and diisopropylethylamine, to obtain protected Goserelin consisting on amino acids having the sequence of: pGlu-His-Trp-Ser-Tyr(Bzl)-D-Ser(tBu)-Leu-Arg(NO2)-Pro-HNNHCONH2 (SEQ. ID. NO. 4).
17. The process of clause 16, further comprising:
   a) reacting the protected Goserelin under hydrogenolysis conditions;
   b) adding MTBE to obtain a precipitate of Goserelin consisting on amino acids having the sequence of: pGlu-His-Trp-Ser-Tyr-D-Ser(tBu)-Leu-Arg-Pro-HNNHCONH2 (SEQ. ID. NO. 4); and
   c) isolating the Goserelin.
18. The process of clause 1, wherein the protected peptide obtained in step d) is protected Triptorelin precursor consisting on amino acids having the sequence of: pGlu-His(Trt)-Trp-Ser(tBu)-Tyr(tBu)-D-Trp-Leu-Arg(Pbf)-Pro-Gly-OH (SEQ. ID. NO. 2).
19. Protected Triptorelin precursor consisting on amino acids having the sequence of: pGlu-His(Trt)-Trp-Ser(tBu)-Tyr(tBu)-D-Trp-Leu-Arg(Pbf)-Pro-Gly-OH (SEQ. ID. NO. 2).
20. The process of clause 18, wherein the amidation in step e) comprises treating the protected peptide with a coupling reagent in the presence of ammonia in DMF and diisopropylethylamine, to obtain protected Triptorelin consisting on amino acids having the sequence of: pGlu-His(Trt)-Trp-Ser(tBu)-Tyr(tBu)-D-Trp-Leu-Arg(Pbf)-Pro-Gly-NH2 (SEQ. ID. NO. 2).
21. The process of clause 20, further comprising:
   a) reacting the protected Triptorelin with TFA solution containing water, TIS and EDT;
   b) adding MTBE to obtain a precipitate of Triptorelin consisting on amino acids having the sequence of: pGlu-His-Trp-Ser-Tyr-D-Trp-Leu-Arg-Pro-Gly-NH2 (SEQ. ID. NO. 2); and
   c) isolating the Triptorelin.
22. The process of clause 1, wherein the protected peptide obtained in step d) is protected Eptifibatide precursor consisting on amino acids having the sequence of: Mpa(Trt)-Har-Gly-Asp(tBu)-Trp-Pro-OH (SEQ. ID. NO. 7).
23. Protected Eptifibatide precursor consisting on amino acids having the sequence of: Mpa(Trt)-Har-Gly-Asp(tBu)-Trp-Pro-OH (SEQ. ID. NO. 7).
24. The process of clause 22, wherein the amidation in step e) comprises treating the protected peptide with a coupling reagent in the presence of Cys(Trt)-NH₂ in DMF, to obtain protected Eptifibatide consisting on amino acids having the sequence of: Mpa-Har-Gly-Asp-Trp-Pro-Cys(Acm)-NH₂ (SEQ. ID. NO. 7).
25. The process of clause 24, further comprising:
   a) reacting the protected Eptifibatide with TFA solution containing water, TIS and EDT;
   b) adding ether to obtain a precipitate of non-cyclic Eptifibatide consisting on amino acids having the sequence of: Mpa-Har-Gly-Asp-Trp-Pro-Cys(Acm)-NH₂ (SEQ. ID. NO. 7);
   c) cyclizing the non-cyclic Eptifibatide; and
   d) isolating Eptifibatide consisting on amino acids having the sequence of: Mpa-Har-Gly-Asp-Trp-Pro-Cys-NH2, (SEQ. ID. NO. 7) cyclic (1-7) disulfide.
26. The process of any of clauses 13, 17, 21, and 25, wherein the isolation is done by precipitation.
27. The process of clause 26, wherein the precipitation is from a solvent selected from the group consisting of: methyl-tert-butyl ether (MTBE), diethyl ether, diisopropylether and mixtures thereof.
28. The process of clause 27, wherein the solvent is mixed with methanol, ethanol or acetonitrile.
29. The process of any of clauses 13, 17, 21, and 25, further comprising:
   a) purifying the peptide selected from the group consisting of: Leuprolide, Goserelin, Triptorelin and Eptifibatide by HPLC chromatography, and simultaneously replacing the counter-ion of the peptide with an acetate to obtain peptide acetate; and
   b) drying the solution of the peptide acetate selected from the group consisting of: Leuprolide acetate, Goserelin acetate, Triptorelin acetate and Eptifibatide acetate.
30. The process of clause 29, wherein the drying is by: lyophilizing or spray drying.
31. Leuprolide acetate contains less than about 0.1 % D-Ser⁴-Leuprolide.
32. Leuprolide acetate contains less than about 0.2% D-His²-Leuprolide.
33. Leuprolide acetate contains less than about 0.1% D-pGlu¹-Leuprolide
34. Leuprolide acetate contains not more than about 0.1 % of any other impurity.
35. Goserelin acetate contains less than about 0.5% of any other impurity.
36. Triptorelin acetate contains less than about 0.5% of any other impurity.
37. A peptide acetate selected from the group consisting of: Leuprolide acetate, Triptorelin acetate, Buserelin acetate, Goserelin acetate, Desmopressin acetate, Calcitonin (salmon) acetate, Lanreotide acetate, Vapreotide acetate, Atosiban acetate, Terlipressin acetate, Felypressin acetate, Ornipressin acetate, Vasopressin acetate, Oxytocin acetate, Sincalide acetate, Enfuvirtide acetate, Exenatide acetate, Eptifibatide acetate, Elcatonin acetate, Porcine Secretin acetate, Human Secretin acetate, and Ziconotide acetate having a purity of at least about 99.0% as determined by HPLC method.
38. Pharmaceutical composition comprising peptide acetate, selected from the group consisting of: Leuprolide acetate, Goserelin acetate, Triptorelin acetate and Eptifibatide acetate, made by the process clause 29, and at least one pharmaceutically acceptable excipient.
39. A process for preparing a pharmaceutical formulation comprising combining the peptide acetate, selected from the group consisting of: Leuprolide acetate, Goserelin acetate, Triptorelin acetate and Eptifibatide acetate, made by the process of clause 29, with at least one pharmaceutically acceptable excipient.
40. Use of peptide acetate, selected from the group consisting of: Leuprolide acetate, Goserelin acetate, Triptorelin acetate and Eptifibatide acetate, made by the process of clause 29, for the manufacture of a pharmaceutical composition.
41. A process for preparing peptide acetate selected from the group consisting of: Leuprolide acetate, Goserelin acetate, Triptorelin acetate and Eptifibatide acetate comprising obtaining a peptide which is a C-terminal amide derivative according to clause 1, and converting the obtained peptide which is a C-terminal amide derivative to peptide acetate selected from the group consisting of: Leuprolide acetate, Goserelin acetate, Triptorelin acetate and Eptifibatide acetate.
42. A process for preparing a pharmaceutical formulation comprising combining the peptide acetate obtained in clause 41, with at least one pharmaceutically acceptable excipient.

## Claims

1. A method of preparing goserelin, comprising:
a) providing amino acid, protected or non-protected, attached in its C-terminal to a super-acid labile resin;
b) coupling said amino acid, with another amino acid, protected or non-protected, in the presence of a coupling reagent;
c) repeating step b) to obtain a peptide, wherein the peptide is protected with at least one protecting group which remains on the peptide upon its cleavage from the resin;
d) cleaving said protected peptide from the resin by admixing with a mild acidic solution; and
e) amidating the protected peptide obtained in step d) with a suitable amine.

2. The process of claim 1, wherein the amidation in step e) is in the presence of a base, preferably diisopropylethylamine

3. The process of any preceding claim, wherein the super-acid labile resin is selected from the group consisting of: chlorotrityl resin, Rink acid resin, NovaSyn TGT resin, and HMPB-AM resin.

4. The process of any preceding claim, wherein the coupling reagent is 2-(1H-benzotriazole-1-yl)-1,1,3,3-tetramethyluronium tetrafluoroborate (TBTU)/N-hydroxybenzotriazole (HoBT).

5. The process of any preceding claim, wherein the mild acidic solution in step d) is a solution comprising about 0.1% to about 5% of TFA in an organic inert solvent or a mixture of acetic acid with trifluoroethanol and DCM.

6. The process of any preceding claim, wherein the protected peptide with the resin obtained in step d) is isolated prior to step e).

7. The process of claim 6, wherein the isolation is by precipitation, crystallization, extraction, or chromatography, preferably the isolation is by precipitation.

8. The process of claim 1, wherein the protected peptide obtained after cleavage from the resin is a protected goserelin precursor consisting of amino acids having the sequence of: pGlu-His-Trp-Ser-Tyr(Bzl)-D-Ser(tBu)-Leu-Arg(NO2)-Pro-OH (SEQ. ID. NO. 4).

9. The process of claim 8, wherein the amidation comprises treating a protected goserelin precursor with a coupling reagent in the presence of semicarbazide hydrochloride in DMF/water and diisopropylethylamine, to obtain a protected goserelin precursor consisting of amino acids having the sequence of: pGlu-His-Trp-Ser-Tyr(Bzl)-D-Ser(tBu)-Leu-Arg(N02)-Pro-HNNHCONH2 (SEQ. ID. NO. 4).

10. The process of claim 9, which further comprises, after amidation, reacting the protected goserelin with 5% Pd/C in DMF; adding MTBE to obtain a precipitate of goserelin consisting of amino acids having the sequence of: pGlu-His-Trp-Ser-Tyr(Bzl)-D-Ser(tBu)-Leu-Arg(N02)-Pro-HNNHCONH2 (SEQ. ID. NO. 4); and isolating goserelin consisting of amino acids having the sequence of: pGlu-His-Trp-Ser-Tyr(Bzl)-D-Ser(tBu)-Leu-Arg(N02)-Pro-HNNHCONH2 (SEQ. ID. NO. 4);

11. A process for preparing goserelin acetate comprising obtaining goserelin according to any one of claims 8 to 10, and converting the obtained goserelin to goserelin acetate.

12. A pharmaceutical composition comprising goserelin acetate prepared according to claim 11 and at least one pharmaceutically acceptable excipient.

13. Goserelin acetate having a purity of at least about 99.0% as determined by HPLC method.

14. Goserelin acetate having less than about 0.5% of any other impurity.
